# EUROPEAN PATENT APPLICATION

(11) **EP 1 113 080 A2**
(43) Date of publication of application: **04.07.2001**
(21) Application number: 00310437.9
(22) Date of filing: 24.11.2000
(51) Int. Cl.: C12Q 1/68

(54) **Personal gene library**

(30) Priority: 01.12.1999 US 168297 P; 09.11.2000 US 708493
(71) Applicant: Wang, Xiao Bing, Lutherville, Maryland 21093 (US); Morisawa, Shinkatsu, Osaka-shi, Osaka (JP)
(72) Inventor: Wang, Xiao Bing, Lutherville, Maryland 21093 (US)
(74) Representative: Lipscombe, Martin John

(57) **Abstract**

The invention is directed to an apparatus comprising a solid surface on which is permanently bound a gene library obtained from an individual.

## Description

### Background of the Invention

The invention relates to an apparatus on which is established a permanent gene library for an individual. The invention also relates to a method for making such an apparatus. Further, the invention relates to a method of using the apparatus for clinical diagnosis or research.

Gray et al (U.S. Patent 5,851,769) relates to a quantitative DNA fiber mapping method. However, the Gray '769 patent does not disclose a method of establishing a permanent gene library on a solid substrate as in the present invention.

Chee et al (U.S. Patent 5,837,832) is directed to making an array of the nucleic acid probes on biological chips. However, Chee '832 does not disclose establishing a permanent gene library for an individual coated or linked to a solid surface carrier, which is then used for clinical diagnosis or research, as in the present invention.

Fodor et al (U.S. Patent 5,445,934) is directed to an array of oligonucleotides on a solid substrate. However, Fodor '934 does not disclose establishing a permanent personal gene library on a solid substrate as in the present invention.

There is a need in the art for a method and apparatus for storing personalized genomic information and detecting possible gene mutations in such apparatus over a prolonged period of time that is simple and cost-effective.

### Summary of the Invention

The present invention has met the hereinbefore described need.

The invention is generally directed to an apparatus comprising a solid surface on which is permanently bound a gene library obtained from an individual.

Another embodiment of the invention is a method for coating or linking a gene or genes obtained from an individual on to the above apparatus by physical, chemical, biochemical, or enzymatic means, wherein the gene or genes are bound permanently to the solid carrier.

In the method above, the gene or genes may comprise one or more chromosomes treated with at least one kind of restriction enzyme. Further, the gene or genes may comprise one or multiple pieces of nucleic acid synthesized from a template of chromosome from an individual. The nucleic acid may be deoxyribonucleic acid or ribonucleic acid. The gene or genes may further comprise oligodeoxyribonucleotide or oligoribonucleotide synthesized from a template of chromosome from an individual.

The invention is also directed to a method for obtaining information from an individual gene structure, comprising performing physical, chemical, biochemical, or enzymatic assays on the above-mentioned apparatus, by detecting the presence or absence of a signal to indicate the presence or absence of a nucleotide base sequence of interest. In this method, the physical, chemical, biochemical, or enzymatic methods may comprise amplification of gene, primer extension, hybridization, sequencing, DNA synthesis, RNA synthesis, oligo DNA or RNA primer synthesis, or protein synthesis methods. In this method, at least one type of nucleotide or amino acid is labeled with detectable marker. The detectable marker may include a radioisotopically labeled moiety, a chromophore, a fluorophore, an enzyme or a detectable protein moiety.

These and other objects of the invention will be more fully understood from the following description of the invention, the referenced drawings attached hereto and the claims appended hereto.

### Brief Description of the Drawings

The present invention will become more fully understood from the detailed description given hereinbelow, and the accompanying drawings which are given by way of illustration only, and thus are not limitative of the present invention, and wherein;

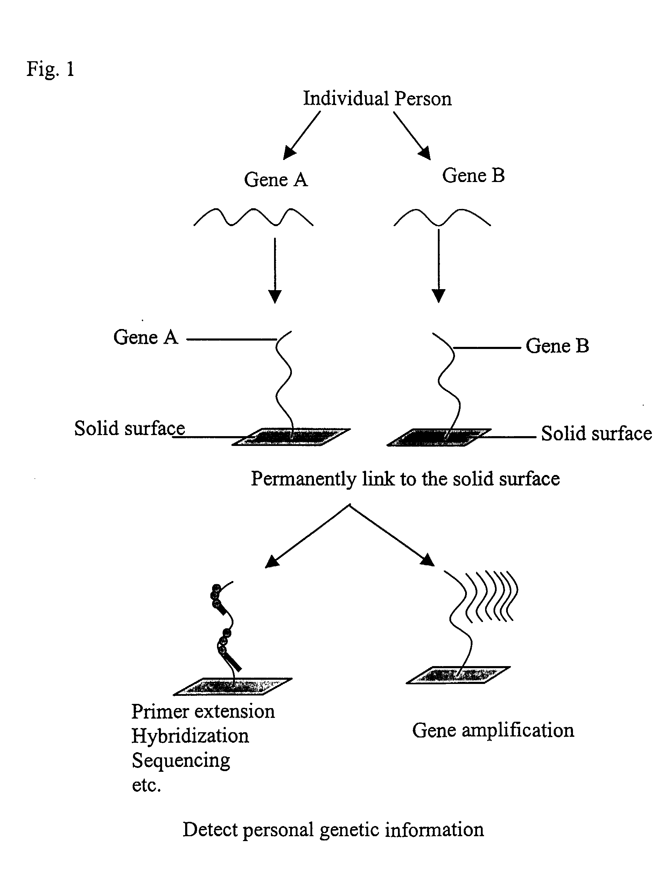

Figure 1 shows a schematic diagram of the process of the present invention for detecting personal genetic information. As shown in Figure 1, an individual's genomic library is permanently linked to a solid surface. Amplification reaction such as polymerase chain reaction (PCR) is carried out on the solid surface to obtain the multiple copy of a specific gene linked on the solid surface. Primer extension, hybridization and sequencing reactions are carried out to detect a particular nucleic acid or nucleotide of interest, if there is any, and otherwise to gain genetic information.

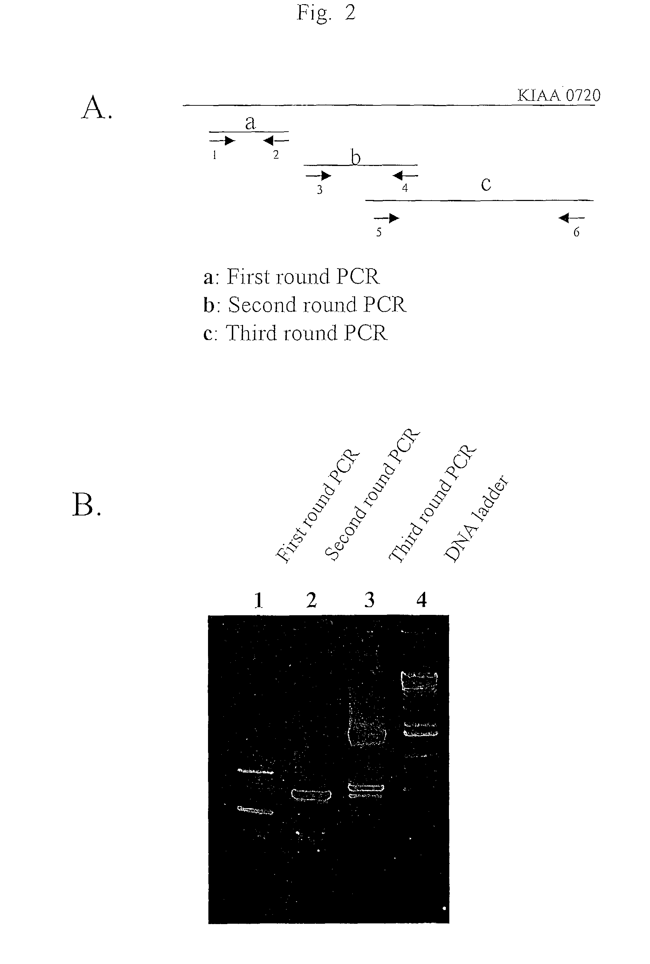

Figure 2 shows PCR amplification of cross-linked sample DNA. A plasmid of rat cDNA clone (similar to human cDNA clone GenBank Accession # KIAA0720) was cross-linked to Nytran membrane (Schleicher & Schuell) and a piece (2x3 mm) of this membrane was amplified by PCR as described in Example 1. The same piece of membrane was repeatedly used for different PCR reaction. Each PCR reaction was designed to amplify a different fragment of the same gene. The Primers used in each PCR is schematically shown in A, and the sequence of each primer is listed in Table 1. In B. Lane 1 is 1 kb DNA marker; lane 2 shows the PCR products from the first round of reactions; lane 3 shows PCR products from the second round; lane 4 shows a PCR products from the third round.

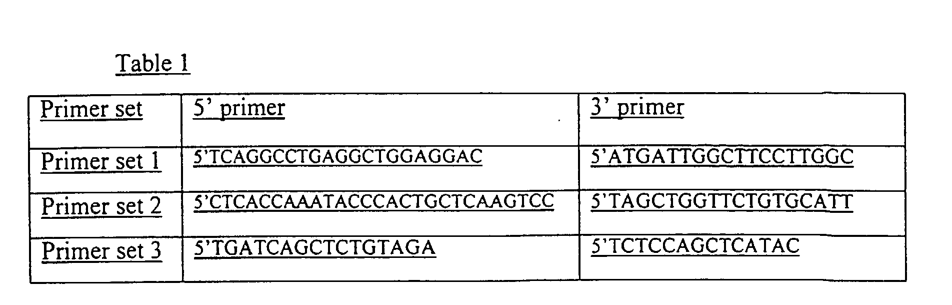

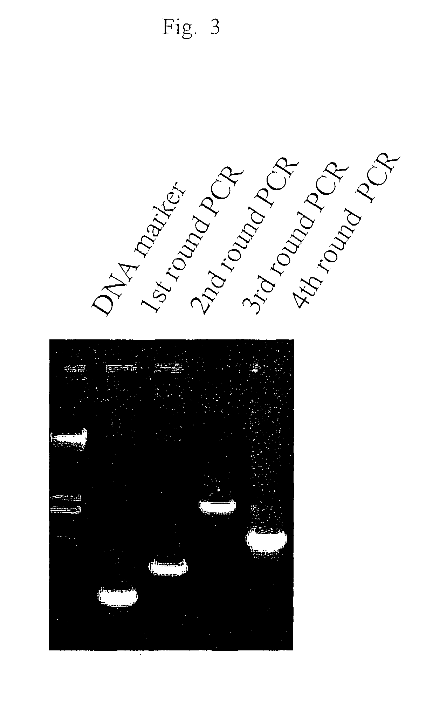

Figure 3 shows PCR amplification of cross-linked human genomic DNA. Human genomic DNA (Clontech CA) was cross-linked to Nytran membrane (Schleicher & Schuell) and a piece (2x3 mm) of this membrane was amplified by PCR as described in Example 1. The same piece of membrane was repeatedly used for different PCR reactions. Each PCR reaction was designed to amplify a different fragment of human beta-actin gene. The sequence of each primer is listed in Table 2. The PCR products were applied to 1% agarose gel. Lane 1 is 1 kb DNA marker; lane 2 shows the PCR products from the first round of reactions; lane 3 shows PCR products from the second round of reactions; lane 4 shows PCR products from the third round of reactions; and lane 5 shows the PCR products from the fourth round of reactions.

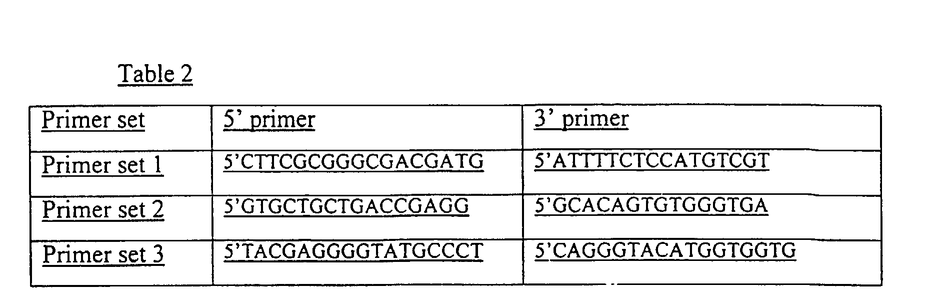

### Detailed Description of the Invention.

The present invention is generally directed to a art for permanently coating or linking a gene or a gene library obtained for an individual to a surface of a solid carrier by physical, chemical, biochemical, or enzymatic means. The invention also relates to an apparatus on which is established a permanent gene library for an individual. The invention also relates to a method for making such a nucleic acid coated or linked apparatus. Further, the invention relates to a method of repeatedly using the nucleic acid coated or linked apparatus for clinical diagnosis or research, such as for detecting genetic markers.

As used herein, the solid carrier that is permanently coated or linked with a gene or genes from an individual is called Gene Library for Individual (GLi). When the solid carrier is coated with a gene or genes from an individual person, it is called Gene Library for Individual Person (GLI*p*). The solid carrier that is coated with a gene or genes from an individual animal is called Gene Library for Individual Animal (GLI*a*). A solid carrier that is coated with a gene or genes for an individual plant is called Gene Library for Individual Plant (GLI*plan).* Further, a solid carrier that is coated with a gene or genes from an individual microorganism such as bacteria, virus, mycoplasma, is called Gene Library for Individual Microorganism (GLI*m*).

The gene obtained from the individual described above can be from one type or multiple types of chromosome. Alternatively, the gene obtained from the individual can be one type or multiple types of chromosome that is digested with at least one type of restriction enzyme.

The gene obtained from the individual, as above can be a piece of DNA fragment of a chromosome that is digested with at least one restriction enzyme. Or, the gene obtained from an individual can be multiple pieces of DNA fragments of a chromosome or chromosomes that are digested with at least one restriction enzyme.

The gene obtained from the individual can be multiple pieces of DNA fragments of multiple or different kinds of chromosomes that are digested with at least one kind of restriction enzyme. The gene from the individual can include a multiple or all of the chromosomes of an individual. Also, the gene from the individual can be multiple or all of the chromosomes from the individual that are digested with at least one kind of restriction enzyme.

The gene from an individual can be one or multiple pieces of DNA that are synthesized from a template of chromosome or chromosomes from the individual. Or, the gene from an individual can be one or multiple pieces of RNA that are synthesized from a template of a chromosome or chromosomes from the individual.

The gene from an individual can be oligodeoxyribonucleotide that is synthesized from a template of chromosome or chromosomes from the individual. Alternatively, the gene obtained from an individual can be an oligoribonucleotide that is synthesized from a template of chromosome or chromosomes from the individual.

In one embodiment of the invention, GLi can be used to check the individual gene or genomic DNA information by physical, chemical, biochemical or enzymatic methods, such as gene amplification, primer extension, hybridization, sequencing, DNA synthesis, RNA synthesis, oligo DNA or oligo RNA primer synthesis, or protein synthesis.

In a preferred embodiment of the invention, the nucleotides or amino acids that are used in the methods outlined above can be labeled with detectable markers. Each of the detectable markers attached to the nucleotides or amino acids can be a radioisotopically labeled moiety, a chromophore, a fluorophore, or moieties to which a radioisotope, a chromophore, fluorophore, enzyme or a protein moiety can be attached.

In another aspect of the invention, the primers or probes that are used to check the individual gene or genomic DNA information can be labeled with detectable markers. The detectable markers attached to the nucleotides or amino acids can be a radioisotopically labeled moiety, a chromophore, fluorophore, or moieties to which a radioisotope, a chromophore, a fluorophore, enzyme or a protein moiety can be attached.

Preferably, the primers or probes used in the method for checking the individual gene or genomic DNA information according to the method of the present invention can be an oligodeoxyribonucleotide, oligoribonucleotide, a fragment of DNA or RNA, or synthetic nucleotide polymer.

As used herein, a "solid surface carrier", "surface solid carrier", "solid carrier" or "solid surface" all refer to a solid phase on which can be permanently bound the nucleic acid obtained from a single individual. The method of linking, crosslinking, or coating can be performed in any manner at all as long as the nucleic acid is able to be detected at a later time by any of the nucleic acid detection methods available.

The nucleic acid so linked or coated to the solid phase is bound permanently. As a result, one of the advantages of having an apparatus of the invention is that a gene test can be performed on the individual from which the gene library is made, numerous times by simply stripping off the hybridized signal-bearing complementary probes, and reusing the apparatus to hybridize with a different genetic probe. The method requires only a one-time withdrawal of blood to make the gene library. Thus, the genetic testing method of the invention does not require withdrawing prohibitive amount of blood from an individual to test for a variety of possible genetic lesions, which is a drawback of the genetic testing protocol currently practiced in the art.

Nucleic acid detection methods are generally known in the art.

The following examples are offered by way of illustration of the present invention, and not by way of limitation.

### EXAMPLES

### Example 1

A plasmid containing a rat cDNA clone (similar to human cDNA clone GenBank Accession # KIAA0720) was selected as a test sample (DNA sample). The DNA sample was diluted with water to final concentration of 1 µg per µl and denatured by heating at 100° C for 3 minutes followed by quenching on ice for 5 minutes. One µl of denatured sample was applied to 2x6 mm Nytran membrane (Schleicher & Schuell) and dried at 80° C for 2 hr to permanently link the sample to the Nytran membrane. A piece (2x2 mm) of DNA sample-coated Nytran membrane was used as template for polymerase chain reaction (PCR). PCR amplification was performed in a total volume of 50 µl in a buffer containing 10 mM Tris-HCl, pH 8.3, 50 mM KCl, 2 mM MgCl₂, 0.2 µmol primers, 20µM of dNTP and 5 units of Taq DNA Polymerase. Thirty cycles of 94° C for 45 s, 55° C for 1 minute and 72° C for 2 minutes were performed in a thermocycler (Perkin Elmer, GeneAmp 9600). After PCR reaction, the same piece of Nytran membrane was removed from the reaction mix, rinsed with water and re-applied to the next PCR reaction. Each PCR was designed to use the same piece of Nytran membrane as template to amplify a different fragment of the DNA sample. Finally, the PCR products are applied to 1% agarose gel and the results are shown in Figure 2.

### Example 2

A commercial human genomic DNA (Clontech, CA) was used as a test sample (DNA sample). The DNA sample (5 µg per µl) was denatured by heating at 100° C for 3 minutes followed by quenching on ice for 5 minutes. Four µl of denatured sample was applied to 2x6 mm Nytran membrane (Schleicher & Schuell) and dried at 80° C for 2 hr to permanently link the sample to the Nytran membrane. A piece (2x3 mm) of DNA sample-coated Nytran membrane was used as the template for polymerase chain reaction (PCR). PCR amplification was performed in a total volume of 50 µl in a buffer containing 10 mM Tris-HCl, pH 8.3, 50 mM KCI, 2 mM MgCl₂, 0.2 µmol primers, 20µM of dNTP and 5 units of Taq DNA Polymerase. Thirty cycles of 94° C for 1 minute, 55° C for 2 minute and 72° C for 2 minutes were performed in a thermocycler (Perkin Elmer, GeneAmp 9600). After the PCR reaction, the same piece of Nytran membrane was removed from the reaction mix, rinsed with water and re-applied for the next round of PCR reactions. Each PCR was designed to use the same piece of Nytran membrane as the template to amplify a different fragment of the DNA sample. Finally, the PCR products were applied to 1% agarose gel and the results are shown in Figure 3.

All of the references cited herein are incorporated by reference in their entirety.

## Claims

1. An apparatus comprising a solid surface on which is permanently bound a gene library obtained from an individual.

2. A method for coating or linking a gene or genes obtained from an individual on to the apparatus of claim 1 by physical, chemical, biochemical, or enzymatic means, wherein the gene or genes are bound permanently to the solid carrier.

3. The method according to claim 2, wherein the gene or genes comprises one or more chromosomes treated with at least one kind of restriction enzyme.

4. The method according to claim 2, wherein the gene or genes comprises one or multiple pieces of nucleic acid synthesized from a template of chromosome or chromosomes from an individual.

5. The method according to claim 4, wherein the nucleic acid is deoxyribonucleic acid or ribonucleic acid.

6. The method according to claim 2, wherein the gene or genes comprises oligodeoxyribonucleotide or oligoribonucleotide synthesized from a template of chromosome or chromosomes from an individual.

7. A method for obtaining information from an individual gene structure, comprising performing physical, chemical, biochemical, or enzymatic assays on the apparatus according to claim 1, by detecting the presence or absence of a signal to indicate the presence or absence of a nucleotide or amino acid sequence of interest.

8. The method according to claim 7, wherein said physical, chemical, biochemical, or enzymatic methods comprise gene amplification, polymerase chain reaction, primer extension, hybridization, sequencing, DNA synthesis, RNA synthesis, oligo DNA or RNA primer synthesis, or protein synthesis methods.

9. The method according to claim 8, wherein at least one type of nucleotide or amino acid labeled with a detectable marker is used for the reaction.

10. The method according claim 9, wherein said detectable marker includes a radioisotopically labeled moiety, a chromophore, a fluorophore, an enzyme or a detectable protein moiety.

11. The method according to claim 2, wherein the gene or genes comprises one or more chromosomes not treated with any restriction enzyme.


SEQUENCE LISTING

<110> Wang, Xiao B.
      Morisawa, Shinkatsu

<120> Personal Gene Library

<130> MJL/c626'1w

<140>
<141>

<160> 12

<170> PatentIn Ver. 2.1

<210> 1
<211> 21
<212> DNA
<213> Artificial Sequence

<220>
<223> Description of Artificial Sequence:
      Oligonucleotide primer

<400> 1
tcaggcctga ggctggagga c                                           21


<210> 2
<211> 18
<212> DNA
<213> Artificial Sequence

<220>
<223> Description of Artificial Sequence:
      Oligonucleotide primer

<400> 2
atgattggct tccttggc                                               18


<210> 3
<211> 27
<212> DNA
<213> Artificial Sequence

<220>
<223> Description of Artificial Sequence:
      Oligonucleotide primer

<400> 3
ctcaccaaat acccactgct caagtcc                                     27

<210> 4
<211> 18
<212> DNA
<213> Artificial Sequence

<220>
<223> Description of Artificial Sequence:
      Oligonucleotide primer

<400> 4
tagctggttc tgtgcatt                                                    18


<210> 5
<211> 16
<212> DNA
<213> Artificial Sequence

<220>
<223> Description of Artificial Sequence:
      Oligonucleotide primer

<400> 5
tgatcagctc tgtaga                                                      16


<210> 6
<211> 14
<212> DNA
<213> Artificial Sequence

<220>
<223> Description of Artificial Sequence:
      Oligonucleotide primer

<400> 6
tctccagctc atac                                                        14


<210> 7
<211> 17
<212> DNA
<213> Artificial Sequence

<220>
<223> Description of Artificial Sequence:
      Oligonucleotide primer

<400> 7
cttcgcgggc gacgatg                                                     17


<210> 8
<211> 16
<212> DNA
<213> Artificial Sequence

<220>
<223> Description of Artificial Sequence:
      Oligonucleotide primer

<400> 8
attttctcca tgtcgt                                                    16


<210> 9
<211> 16
<212> DNA
<213> Artificial Sequence

<220>
<223> Description of Artificial Sequence:
      Oligonucleotide primer

<400> 9
gtgctgctga ccgagg                                                    16


<210> 10
<211> 15
<212> DNA
<213> Artificial Sequence

<220>
<223> Description of Artificial Sequence:
      Oligonucleotide primer

<400> 10
gcacagtgtg ggtga                                                     15


<210> 11
<211> 17
<212> DNA
<213> Artificial Sequence

<220>
<223> Description of Artificial Sequence:
      Oligonucleotide primer

<400> 11
tacgaggggt atgccct                                                   17

<210> 12
<211> 17
<212> DNA
<213> Artificial Sequence

<220>
<223> Description of Artificial Sequence:
      Oligonucleotide primer

<400> 12
cagggtacat ggtggtg                                                17